# EUROPEAN PATENT APPLICATION

(11) **EP 4 537 894 A1**
(43) Date of publication of application: **16.04.2025**
(21) Application number: 23203450.4
(22) Date of filing: 13.10.2023
(51) Int. Cl.: A61N 5/06

(54) **A WEARABLE LIGHT THERAPY DEVICE**

(71) Applicant: Motherson Innovations Company Ltd., London EC2N 2AX (GB)
(72) Inventor: JONES, Matthew, Lonsdale, South Australia 5160 (AU); LI, James, Lonsdale, South Australia 5160 (AU); GREEN, Anthony, Lonsdale, South Australia 5160 (AU); GOYAL, Nitin, Gurgaon, Haryana 122002 (IN); SINGH, Anchita, Gurgaon, Haryana 122002 (IN); LACK, Leon, Adelaide S.A. 5001 (AU)
(74) Representative: Isarpatent

(57) **Abstract**

A wearable light therapy device for administering light into the eyes of a wearer, and a method of managing circadian rhythm disorders, including sleep onset insomnia and early morning awakening insomnia, jet-lag and shift work fatigue, the light therapy device comprising a pair of side supporting arms with a bridging component therebetween, wherein each supporting arm comprises a free end and a hinged end, the free end being for mounting upon a respective ear of a wearer, and the hinged end being pivotally attached to one end of the bridging component; and characterised in that the bridging component comprises a raised nose portion configured between lower opposed lateral portions, each lateral portion having at least one light aperture for directing light from at least one light source into the eyes of a wearer, the nose portion being mountable upon the nose of a wearer during use such that the lateral portions are generally below the eyes of the wearer.

## Description

### Field of the Invention

This invention relates to wearable devices suitable for providing a wearer with visual light stimulation for the management of circadian rhythm disorders including sleep onset insomnia and early morning awakening insomnia, jet-lag, shift work fatigue, and other conditions requiring the re-timing of human circadian rhythms (the 24-hour body clock).

### Background of the Invention

The timing of the body's circadian rhythms very strongly determines our best times to sleep and to be awake and alert. The anatomical pathways from the retina to the brain and the hormonal and biological mechanisms underlying the biological clock resetting capacity of bright light are known. It was first demonstrated in 1980 that bright exposure could have a significant and direct impact on human physiological brain function. Since then, the use of bright light stimulation at appropriate times in a 24-hour period has been shown to change the timing of the body's endogenous circadian rhythms. In an individual who sleeps from 11pm to 7am, stimulation in the evening, up to about 4am, delays circadian rhythms, whilst stimulation in the morning between 5am and 9am phase advances rhythms to an earlier time.

Inappropriate timing of circadian rhythms is implicated in several sleep/wake and mood disorders. Delayed and advanced sleep phase syndromes, which adversely affects the sleep and alertness of their sufferers for several hours each day, arise from delayed and advanced circadian rhythms. Research has shown that sleep onset insomnia and early morning awakening insomnia can be caused by delayed and advanced circadian rhythms respectively.

Several methods have been proposed to deliver visual light stimulation for the adjustment of circadian rhythms. One approach utilises light boxes containing high intensity fluorescent light fixtures. However, these light boxes are large and require domestic high voltage power sources. Users must maintain visual contact with the light box when it is in use and consequently are restricted in their movement.

Another method is the use of a head mounted light visor as disclosed in US patent 5447528. The light visor mounted at the forehead, well above the normal direction of gaze, is unlikely in most applied settings to stimulate the visual system with adequate light intensity to effect re-timing of the body clock. No study has confirmed their efficacy for this purpose.

Two further methods are described in US patent publications 2006/0136018 and 2013/0253619, both of which utilise a traditional pair of spectacles, with eyeglass lenses and frames, where the eyeglass lenses have mounted thereto, or have integral therewith, light sources able to direct light into the retina of a wearer. The devices of both prior art publications are relatively cumbersome and require the user to be comfortable wearing spectacles, which can be difficult at night and in some circumstances, and for individuals who do not normally wear spectacles, can be a disincentive to attempt the light therapy.

The present invention seeks to provide an improved light therapy device for management of circadian rhythm disorders including sleep onset insomnia and early morning awakening insomnia, jet-lag and shift work fatigue, together with an improved method for the management of such circadian rhythm disorders. In at least one form, this is ideally achieved by administering sufficient intensity of an appropriate wavelength to effect re-timing of the human body clock, in a convenient and effective manner.

Before turning to a summary of the invention, it should be appreciated that reference to any prior art in this specification is not, and should not be taken as, an acknowledgment or any form of suggestion that this prior art is either well known or forms part of the common general knowledge in any country.

Also, the following description will use directional terms such as downward and downwardly, upward and upwardly, lower and upper, and above and below, which will be used with reference to the light therapy device of the present invention being worn on the face of a wearer with the wearer standing upright. Further, there will also be references to inner and outer, and inwardly and outwardly, which will be references again made with respect to the face of a wearer, with inner and inwardly being a position between the device and a wearer's face, or a direction towards the wearer's face.

### Summary of the Invention

The present invention provides a wearable light therapy device for administering light into the eyes of a wearer, the light therapy device comprising a pair of side supporting arms with a bridging component therebetween, wherein each supporting arm comprises a free end and a hinged end, the free end being for mounting upon a respective ear of a wearer, and the hinged end being pivotally attached to one end of the bridging component;
characterised in that the bridging component comprises a raised nose portion configured between lower opposed lateral portions, each lateral portion having at least one light aperture for directing light from at least one light source into the eyes of a wearer, the nose portion being mountable upon the nose of a wearer during use such that the lateral portions are generally below the eyes of the wearer.

The present invention also provides a wearable light therapy device for use in the management of circadian rhythm disorders, including sleep onset insomnia and early morning awakening insomnia, jet-lag and shift work fatigue, the light therapy device comprising:
a pair of side supporting arms with a bridging component therebetween, wherein each supporting arm comprises a free end and a hinged end, the free end being for mounting upon a respective ear of a wearer, and the hinged end being pivotally attached to one end of the bridging component;
characterised in that the bridging component comprises a raised nose portion configured between lower opposed lateral portions, each lateral portion having at least one light aperture for directing light from at least one light source into the eyes of a wearer, the nose portion being mountable upon the nose of a wearer during use such that the lateral portions are generally below the eyes of the wearer.

The present invention also provides a method of managing circadian rhythm disorders, including sleep onset insomnia and early morning awakening insomnia, jet-lag and shift work fatigue, the method including:
a) placing a light therapy device on the face of a wearer, the light therapy device comprising a pair of side supporting arms with a bridging component therebetween, wherein each supporting arm comprises a free end and a hinged end, the free end being for mounting upon a respective ear of the wearer, and the hinged end being pivotally attached to one end of the bridging component, the bridging component comprising a raised nose portion configured between lower opposed lateral portions, each lateral portion having at least one light aperture for directing light from at least one light source into the eyes of the wearer, the nose portion being mountable upon the nose of the wearer during use such that the lateral portions are generally below the eyes of the wearer; and
b) administering light from the light apertures into the eyes of the wearer.

In a preferred form, the light therapy device of the present invention does not include eyeglass lenses of the type used in spectacles and sunglasses, nor therefore an eyeglass lens frame. The supporting arms and the bridging component thus together form a single member that will lie across a wearer's face, supported on the wearer's ears and nose, with the lateral portions of the bridging component generally below the eyes of the wearer. In this form, there is thus no straight-ahead visual obstacle for the wearer when wearing the light therapy device.

The nose portion of the light therapy device is a raised portion of the bridging component, preferably positioned centrally between the lateral portions. In one form, the nose portion will be an inverted U-shaped portion configured to fit snugly over the bridge of the nose of a wearer, like the traditional bridge of a normal pair of spectacles, with the lateral portions of the bridging component of the device extending laterally away from the (notionally) free ends of the arms of the inverted U.

In a preferred form, the nose portion will include mounting regions for nose pads, such as silicon nose pads that might be small, medium and large nose pads, to assist with fitting the device snugly to the nose of a wearer with a small, medium or large nose. The nose portion, including nose pads if present, will preferably be sized and profiled to fit snugly and smoothly between the lateral portions of the bridging component to leave no obvious transition between the nose portion and the lateral portions, and so as to streamline the appearance of the bridging component to present the bridging component as being a single continuous member.

The nose pads may be screw-in, push-in, clip-on or moulded in-situ nose pads, and the nose pads may be fixed or may be adjustable. In a preferred form, nose pads may clip onto a moulded extension of the nose portion. Preferably any nose pads that are included will be capable of being removed to be replaced or cleaned. Preferably the nose pads will also be profiled to fit snugly and smoothly within or upon the nose portion, again to assist with streamlining the overall appearance of the bridging component and thus the light therapy device.

In a preferred form, the light sources are LEDs mounted within each lateral portion behind a light aperture, such as two LEDs mounted within each lateral portion, each behind a respective light aperture, each light aperture being configured for directing light from its LED into an eye of the wearer. Each light aperture will preferably include a diffusive window through which light passes before entering the wearer's eyes, ideally to dull the intensity of the light.

In another form, the device may include a light pipe (sometimes referred to as a "light tube") within the bridging component that provides light from a light source mounted in one or both of the supporting arms, to the light apertures of the lateral portions of the bridging component, into the eyes of a wearer during use. Such a light pipe may for instance be a fibre-optic cable or a custom moulded pathway from a light source, through the bridging component, to the light apertures. With this in mind, a light pipe will direct illumination from, for example, LEDs on a circuit board mounted in a supporting arm to the light apertures and will have a shape that uses curving bends that can be provided by a fibre-optic cable, or sharp prismatic folds which reflect off angled corners that might be provided by a custom moulded pathway.

Ideally, the light projected from the light apertures will be from generally below the eyes of the wearer. In this respect, references in this specification to "the bridge of the nose a wearer" and "generally below the eyes of the wearer" should be understood to be approximations of the likely and ideal positioning of the light therapy device of the present invention upon the face of a wearer. As would be expected, and similarly to the wearing of spectacles, a user might prefer the light therapy device to sit high on the bridge of their nose or further down their nose more towards the tip of their nose. This will result in the lateral portions of the bridging component of the device, and thus the light apertures thereof, adopting slightly different positioning with respect to the user's eyes.

In a preferred form, the vertical distance from the lateral portions to the top of the nose portion of the device will at least be such that the lateral portions will always be generally below the user's eyes when the device is positioned as high upon the bridge of the user's nose as reasonably likely - this will be referred to hereafter as the maximum height position. In this respect, it is preferred that, at the maximum height position for the device in use, the upper surface of the lateral portions will be at or below the lower eyelids of the user.

In this form, with the light apertures of the lateral portions being oriented to project generally upwardly, but inclined towards the eyes of the wearer, light projected into the user's eyes will be projected from generally below the user's eyes, at an angle into the eyes of a wearer. And as mentioned above, with the light therapy device not including eyeglass lenses, the light projected does not pass through an eyeglass lens.

With respect to the supporting arms of the device, which are the equivalent of the component in spectacles often referred to as "temples", in a preferred form one or both of the supporting arms will contain all of the supporting electronics desired for operation of the device. The supporting electronics may include a circuit board containing, amongst other things, a processor, memory, capacitors, a rechargeable battery, power terminals, a USB connector, light sensors, light sources such as LEDs and wireless communication electronics such as a Bluetooth connection module.

The processor may be used, for example, to run programmed light therapy schedules stored in memory, to analyse light sensor data and determine a unique light therapy schedule based on the wearer's exposure to ambient light, to evaluate manual changes to a programmed light therapy schedule and provide compensating adjustments, and to analyse light source and light sensor data to detect device failures, amongst other things. The memory may be used, for example, to store preprogrammed light therapy schedules, to store data captured by light sensors, to store actual light therapy dates, times, durations and intensities, and to store data related to light source and light sensor operation in order to detect device failures, amongst other things.

When the supporting electronics of the light therapy device of the present invention includes wireless communication electronics, such as the Bluetooth module, an external device provided with a processor may be utilised with the light therapy device, by way of a computer program which, when in use, causes the processor to control the light source in the light therapy device according to a predetermined program, the desired program ideally being selectable by the wearer. The external device is preferably a portable apparatus such as a smartphone/tablet or the like, and the computer program can be an application (an App) that is installable on the smartphone/tablet, which app can communicate wirelessly with the light therapy device via Bluetooth technology, for example to periodically start or to end a light session according to a predetermined or selected program.

In a preferred form, an App which can connect with the device might offer different programmed schedules to the wearer for the use of the device. The schedules may suggest the time and duration the device should be worn based on the need of the wearer. Different programs will ideally deal with late and early onset of sleep, shift work, and jet lag. The App may also collect information from the device when the device is turned on and off, to provide the wearer with reports on how they are adhering to a suggested schedule. The App may also provide an ability to connect with other Apps (such as Apple Health and FitBit (Google)), so that sleep data can be uploaded for the purpose of reporting on the App.

In relation to the light projected into the eyes of the wearer, the light therapy device of the present invention will preferably include one or more light sources, such as the LEDs mentioned above, which will preferably emit green light at wavelengths of 475nm to 525nm, more preferably at 490nm to 51 0nm, and at 300 to 400 lux.

In one form, the different areas of a wearer's retinas that are illuminated by the light sources includes areas of the retina which are outside of the macula. Thus, in one form, the light sources (which will generally be referred to hereafter simply as LEDs) are configured and directed such that different areas of the wearer's retinas are illuminated to exclude the macula. These different areas are located in what will subsequently be referred to in this specification as the "peripheral retina". Moreover, for the purposes of this description, references to the term "exclude the macula", when used in relation to the term "illumination of the different areas", is to be understood as meaning that the macula is not directly illuminated by the projected light of an LED. However, it will be appreciated that the macula may be indirectly illuminated by the projected light of an LED because of "intraocular scattering".

Illumination of areas of the retina which exclude the macula is particularly beneficial because it avoids illumination of the macula itself. As will be appreciated, the macula is that part of the retina which is responsible for sharp straight-ahead vision (necessary for functions such as reading and driving a car). Thus, not illuminating the macula reduces the extent to which the light therapy device of the present invention interferes with normal vision. Moreover, because the peripheral retina contains a high expression of photoreceptors which govern circadian photoreception, the illumination of the peripheral retina is particularly advantageous for effecting re-timing of the human body clock.

A set of blue-light photoreceptors (the CRY1 and CRY2 cryptochromes) are localised in the inner nuclear layer (INL) and ganglion cell layer (GCL) of the peripheral retina. Accordingly, in one form of the present invention, LEDs having a peak intensity at a wavelength of about 470 nm to 480 nm (that is, a wavelength to which the photoreceptors are responsive) are preferably used. In this form, and when combined with configuring LEDs to allow different areas of the peripheral retina to be illuminated, photoreceptors which are specifically responsive to the projected light to effect re-timing of the human body clock can be illuminated.

Preferably, each LED will project a spreading beam shaped light output (such as a conical beam) and will be positioned in use adjacent a surface of each eye to provide an illuminated region on the surface thereof. Preferably, the illuminated region will be a disc-shaped region having an area which permits a portion of the light to pass through the pupil of the respective eye to illuminate a respective area of the peripheral retina. In one form, the disc shaped region may have a diameter that is substantially the same diameter as the diameter of the iris.

The positioning of LEDs adjacent to the surface of a wearer's eye, combined with the geometry of the beam shaped light output, may provide an illuminated region which permits areas of the peripheral retina to be illuminated throughout a range of movement of the eye.

In one form, each LED will be positioned adjacent a surface of each eye and arranged so that the light output from each LED of a pair of LEDs combines to provide a substantially uniform irradiance of substantially the entire area of the pupil of a respective eye. Indeed, in one embodiment the substantially uniform irradiance is provided to allow for a range of movement of the respective pupil. In this respect, reference to the term "a range of movement", where used throughout this specification in relation to the pupil, is to be understood to be reference to an area within which the pupil may move because of dilation and/or rotation of the eye.

Preferably, the distance from the LEDs to the corneal surface of the eyes of the wearer will be between 10 mm and 15 mm. In this form, the disc shaped region will preferably subtend between 15 degrees and 20 degrees. In a further preferred arrangement, LEDs will be positioned about 15 degrees away from the forward direction of gaze of the wearer both to the nasal and the lateral visual field.

In one specific form, the effective intensity of the LEDs at the cornea surface (measured photometrically) will be in the range of 1000 lux to 4000 lux. More precisely, in terms of the electromagnetic energy incident at the pupil of the eye of a wearer the effective intensity will preferably be between 100 uw/cm² and 400 uw/cm².

Preferably, LEDs will be arranged such that the respective different areas of the peripheral retina which are illuminated by a pair of LEDs will include a nasal area and a lateral area. Thus, in one form, one LED from a pair of LEDs will be able to illuminate the nasal area of a respective peripheral retina and the other LED of that pair will be able to illuminate the lateral area.

Each viewing zone may have a width in the range of 15 mm to 20 mm as measured between the LEDs. Such an arrangement may be useful since it allows the LEDs to project light via the pupil to illuminate areas of the peripheral retina which are not able to be illuminated when the LEDs are spaced further apart.

In one form, pairs of LEDs may be arranged so that the LEDs are collinear. In this form, the collinear arrangement of LEDs may be located along a horizontal axis which is located below the horizontal centreline of the wearer's monocular visual field when the wearer adopts a forward direction of gaze (that is, when the wearer looks "straight ahead").

When the LEDs are located below the horizontal centreline of the wearer's monocular visual field, the LEDs may be arranged to project light at respective upper areas of the peripheral retina. Thus, one LED of each pair may illuminate an upper nasal area of the peripheral retina whilst the other LED may illuminate the upper lateral area of the peripheral retina. In this way, obstruction of the central portion of each monocular visual field is reduced as compared to a case where the collinear arrangement of LEDs is arranged on the horizontal centreline of the wearer's monocular visual field.

In one form, the collinear arrangement of the LEDs is ideally such that the LEDs are located about 15 degrees below the horizontal centreline of the wearer's monocular visual field. This arrangement has been found to provide a comfortable balance between effective illumination of the wearer's retina and reduction in obstruction of the wearer's monocular visual field.

### Brief Description of Drawings

Having briefly described the general concepts involved with the present invention, a preferred embodiment of a light therapy device will now be described that is in accordance with the present invention. However, it is to be understood that the following description is not to limit the generality of the above description.

In the drawings:
Figure 1 is a general illustration of a light therapy device according to a preferred embodiment, being worn on the face by a wearer;
Figure 2 is an isometric view from the front and above of the light therapy device of Figure 1;
Figure 3 is an isometric view of the light therapy device of Figure 1 viewed from behind and above;
Figure 4 a view of the light therapy device of Figure viewed from behind;
Figure 5 is a view of the light therapy device of Figure 1 viewed from above; and
Figure 6 is a schematic isometric view of the light therapy device of Figure 1 in a disassembled state.

### Detailed Description of Preferred Embodiment

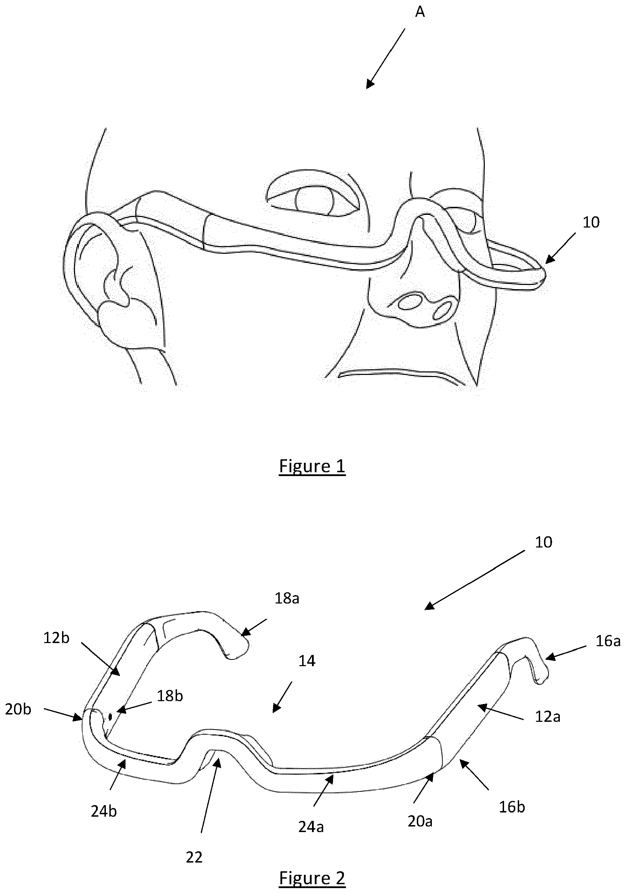

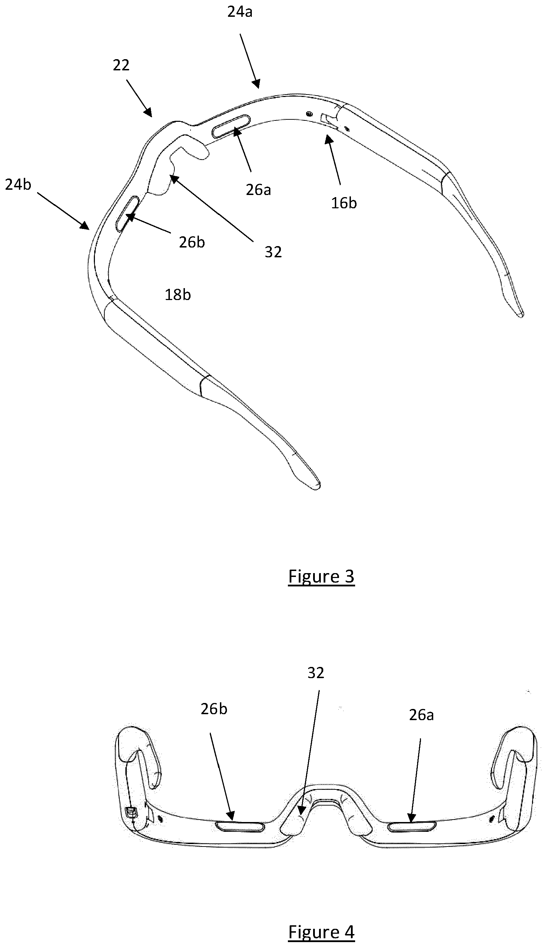

Illustrated in Figures 1 to 5 is a wearable light therapy device 10 for administering light into the eyes of a wearer A (only evident in Figure 1). The device includes a pair of side supporting arms (12a, 12b) with a bridging component 14 therebetween. Each supporting arm (12a, 12b) has a free end (16a, 16b) and a hinged end (18a, 18b), the free ends (16a, 16b) being for mounting upon a respective ear of the wearer A, while the hinged ends (18a, 18b) re pivotally attached to a respective end (20a, 20b) of the bridging component 14.

The bridging component 14 has a raised nose portion 22 configured between lower opposed lateral portions (24a, 24b) of the bridging component 14. Each lateral portion includes, in this embodiment, a single light aperture (26a, 26b) for directing light from, in this embodiment, two light sources each (not shown) into the eyes of the wearer A. Ideally, each light source is a light emitting diode (LED), which will be further described below.

The nose portion 22 is mountable upon the nose of the wearer A during use such that the lateral portions (24a, 24b) are generally below the eyes of the wearer A.

It is thus apparent that the light therapy device 10 does not include eyeglass lenses of the type used in spectacles and sunglasses, nor therefore an eyeglass lens frame. Also, the supporting arms (12a, 12b) and the bridging component 14 together form a single member that lies across a wearer's face, as can be seen in Figure 1, supported on the wearer's ears and nose, with the lateral portions (24a, 24b) of the bridging component 14 generally below the eyes of the wearer A. There is thus no straight-ahead visual obstacle for the wearer A when wearing the light therapy device 10.

In this embodiment, the nose portion 22 is a raised portion of the bridging component 14, positioned centrally between the lateral portions (24a, 24b). The nose portion 22 is an inverted U-shaped portion configured to fit snugly over the bridge of the nose of a wearer A, like the traditional bridge of a normal pair of spectacles, with the lateral portions (24a, 24b) extending laterally away from the nose portion 22, and specifically the notionally free ends of the arms of the inverted U.

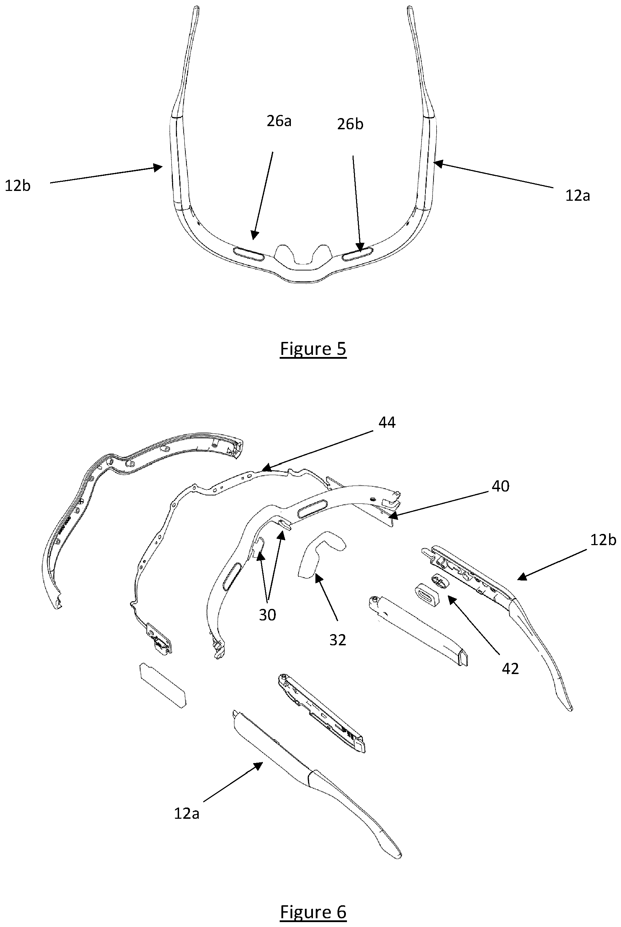

The nose portion 22 will includes moulded mounting extensions 30 for a nose pad 32, illustrated in the disassembled view of Figure 6, in this embodiment being a silicon nose pad that clips onto, and is detachable from, the mounting extensions 30.

In this embodiment, LEDs are mounted within each lateral portion (24a, 24b) behind a light aperture (26a, 26b), such as two LEDs mounted within behind each light aperture (26a, 26b), each light aperture (26a, 26b) being configured for directing light from its LEDs into an eye of the wearer A. Each light aperture (26a, 26b) also includes a diffusive window (not shown) through which light passes before entering the wearer's eyes.

Referring now to Figure 6, which shows the device 10 in a disassembled state, the supporting arms (12a,12b) in this embodiment contain all of the supporting electronics desired for operation of the device 10. The nature of the supporting electronics will be understood by a skilled addressee and for example will normally include a circuit board (such as the circuit board 40 shown in Figure 6) containing, amongst other things, a processor, memory, capacitors, a rechargeable battery, power terminals, a USB connector, light sensors, switches (such as switch 42), and wireless communication electronics such as a Bluetooth connection module. LEDs (not shown here) may then be mounted in an appropriate location on the conductive strip 44.

In relation to the use of the device 10 for the management of circadian rhythm disorders, including sleep onset insomnia and early morning awakening insomnia, jet-lag and shift work fatigue, reference is made to the management theories and regimes described in the journal article, Wright et al; "Light Emitting Diodes Can Be Used to Phase Delay the Melatonin Rhythm"; J. Pineal Res. V 31; 2001; pp 350-355, the entire content of which is incorporated herein by reference in order to assist the skilled addressee to put this aspect of the present invention into practice over the full scope of the claims without an undue burden of trial and experiment, or the need for further invention.

In conclusion, it must be appreciated that there may be other variations and modifications to the configurations described herein which are also within the scope of the present invention.

## Claims

1. A wearable light therapy device for administering light into the eyes of a wearer, the light therapy device comprising a pair of side supporting arms with a bridging component therebetween, wherein each supporting arm comprises a free end and a hinged end, the free end being for mounting upon a respective ear of a wearer, and the hinged end being pivotally attached to one end of the bridging component;
**characterised in that** the bridging component comprises a raised nose portion configured between lower opposed lateral portions, each lateral portion having at least one light aperture for directing light from at least one light source into the eyes of a wearer, the nose portion being mountable upon the nose of a wearer during use such that the lateral portions are generally below the eyes of the wearer.

2. A wearable light therapy device for use in the management of circadian rhythm disorders, including sleep onset insomnia and early morning awakening insomnia, jet-lag and shift work fatigue, the light therapy device comprising:
a pair of side supporting arms with a bridging component therebetween, wherein each supporting arm comprises a free end and a hinged end, the free end being for mounting upon a respective ear of a wearer, and the hinged end being pivotally attached to one end of the bridging component;
**characterised in that** the bridging component comprises a raised nose portion configured between lower opposed lateral portions, each lateral portion having at least one light aperture for directing light from at least one light source into the eyes of a wearer, the nose portion being mountable upon the nose of a wearer during use such that the lateral portions are generally below the eyes of the wearer.

3. A device according to claim 1 or claim 2, **characterised in that** the device does not include eyeglass lenses nor an eyeglass lens frame.

4. A device according to claim 1 or claim 2, **characterised in that** the supporting arms and the bridging component together form a single member that will lie across a wearer's face in use, supported on the wearer's ears and nose, with the lateral portions of the bridging component generally below the eyes of the wearer.

5. A device according to any one of claims 1 to 4, **characterised in that** the nose portion is an inverted U-shaped portion configured so as to fit snugly over the bridge of the nose of a wearer, with the lateral portions extending laterally away from free ends of arms of the inverted U.

6. A device according to claim 5, wherein the nose portion is sized and profiled so as to fit snugly and smoothly between the lateral portions of the bridging component so as to leave no obvious transition between the nose portion and the lateral portions, and so as to streamline the appearance of the bridging component to present the bridging component as being a single continuous member.

7. A device according to any one of claims 1 to 6, **characterised in that** the nose portion includes mounting regions for nose pads that assist with fitting the device snugly to the nose of a wearer, the nose pads preferably being screw-in, push-in, clip-on or moulded in-situ nose pads, and being either fixed, removable or adjustable.

8. A device according to any one of claims 1 to 7, **characterised in that** the light sources are LEDs mounted within each lateral portion behind a light aperture, such as two LEDs mounted within each lateral portion, each behind a respective light aperture, each light aperture being configured for directing light from its LED into an eye of the wearer.

9. A device according to any one of claims 1 to 8, **characterised in that** each light aperture includes a diffusive window through which light passes before entering the wearer's eyes to dull the intensity of the light.

10. A device according to any one of claims 1 to 9, **characterised in that** a vertical distance from the lateral portions to the top of the nose portion will at least be such that the lateral portions, in use, will be generally below the user's eyes.

11. A device according to any one of claims 1 to 10, **characterised in that** the one or more light sources emit green light at wavelengths of 475nm to 525nm, and preferably at 490nm to 510nm.

12. A method of managing circadian rhythm disorders, including sleep onset insomnia and early morning awakening insomnia, jet-lag and shift work fatigue, the method including:
a) placing a light therapy device on the face of a wearer, the light therapy device comprising a pair of side supporting arms with a bridging component therebetween, wherein each supporting arm comprises a free end and a hinged end, the free end being for mounting upon a respective ear of the wearer, and the hinged end being pivotally attached to one end of the bridging component, the bridging component comprising a raised nose portion configured between lower opposed lateral portions, each lateral portion having at least one light aperture for directing light from at least one light source into the eyes of the wearer, the nose portion being mountable upon the nose of the wearer during use such that the lateral portions are generally below the eyes of the wearer; and
b) administering light from the light apertures into the eyes of the wearer.

13. A method according to claim 12, **characterised in that** the one or more light sources emit green light at wavelengths of 475nm to 525nm, and preferably at 490nm to 510nm.

14. A method according to claim 12 or claim 13, **characterised in that** a vertical distance from the lateral portions to the top of the nose portion will at least be such that the lateral portions, in use, will be generally below the wearer's eyes.

15. A method according to claim 14, **characterised in that**, with the light apertures of the lateral portions oriented to project generally upwardly, but inclined towards the eyes of the wearer, light projected into the wearer's eyes is projected from generally below the wearer's eyes, at an angle into the eyes of the wearer.
